# EUROPÄISCHE PATENTSCHRIFT

(11) **EP 2 170 494 B1**
(45) Veröffentlichungstag und Bekanntmachung des Hinweises auf die Patenterteilung: **17.08.2011**
(21) Anmeldenummer: 08774160.9
(22) Anmeldetag: 20.06.2008
(51) Int. Cl.: B01J 8/00, B01J 8/02, B01D 3/00, C10G 49/00

(54) **REAKTOR ZUR DURCHFÜHRUNG EINER DREIPHASENREAKTION EINER FLÜSSIGEN UND EINER GASFÖRMIGEN PHASE AN EINEM KATALYSATORFESTBETT**
REACTOR FOR PERFORMING A THREE-PHASE REACTION OF A FLUID AND A GASEOUS PHASE ON A PACKED BED CATALYST
RÉACTEUR EN VUE DE LA RÉALISATION D'UNE RÉACTION TRIPHASIQUE ENTRE UNE PHASE LIQUIDE ET UNE PHASE GAZEUSE SUR UN LIT FIXE DE CATALYSEUR

(30) Priorität: 21.06.2007 US 945461 P
(43) Veröffentlichungstag der Anmeldung: 07.04.2010
(73) Patentinhaber: BASF SE, 67056 Ludwigshafen (DE)
(72) Erfinder: BECHTEL, Marcus, 69123 Heidelberg (DE); HEPFER, Benjamin, 68163 Mannheim (DE); WILLE, Michael Hyatt Regency Xian,, 710001 Xian (CN); DAISS, Andreas, 67146 Deidesheim (DE); GEIER, Reiner, 68199 Mannheim (DE); ISELBORN, Stefan, 67227 Frankental (DE); SAUTER, John, Bloomfield, NJ 07003-3002 (US)
(86) Internationale Anmeldenummer: PCT/EP2008/057832
(87) Internationale Veröffentlichungsnummer: WO 2008/155399

(56) Entgegenhaltungen:
- GB-A- 1 274 327
- US-A- 4 808 350
- US-A- 5 232 283
- US-A- 5 484 578
- US-A1- 2006 163 758
- US-B1- 6 669 915

## Beschreibung

Die Erfindung betrifft einen Reaktor zur Durchführung einer Dreiphasenreaktion einer flüssigen und einer gasförmigen Phase an einem Katalysatorfestbett mit einer Misch- und Verteileinrichtung für die flüssige und die gasförmige Phase, ein Verfahren zur Durchführung von Dreiphasenreaktionen sowie eine Verwendung.

Dreiphasenreaktionen einer flüssigen Phase und einer gasförmigen Phase an einem Katalysatorfestbett sind in der chemischen Verfahrenstechnik häufig, wobei oft eine Betriebsweise mit einem oder mehreren horizontal in einem vertikal aufrecht stehenden Reaktor angeordneten Katalysatorfestbetten gewählt wird, über die die flüssige und die gasförmige Phase geleitet werden. Umsatz und Selektivität der ablaufenden Reaktionen hängen neben der Reaktionskinetik insbesondere von der Hydrodynamik des Reaktors ab. Dafür ist ein gleichmäßiger Reaktionsfortschritt erforderlich, der wiederum voraussetzt, dass sowohl das Gas als auch die Flüssigkeit, das heißt beide Reaktionspartner, sowohl in radialer als auch in axialer Richtung im Reaktor optimal möglichst gleichförmig verteilt sind. Dies ist insbesondere in großtechnischen Reaktoren mit entsprechend großen Abmessungen problematisch.

Dreiphasenreaktoren werden zudem meistens adiabatisch betrieben, das heißt die Temperatur ändert sich mit fortschreitendem Umsatz in Folge der freiwerdenden oder aufgenommenen Reaktionswärme, die mangels externen Wärmeaustauschs entsprechend steigt oder sinkt. Um eine homogene Temperaturverteilung, ohne Hotspots, und somit ohne entsprechende nachteilige Effekte, insbesondere auf die Katalysatorstandzeit, auf Umsatz und Selektivität, zu erreichen, ist ebenfalls eine gleichmäßige Verteilung der Edukte wünschenswert.

Es wurden daher in der chemischen Verfahrenstechnik eine Vielzahl von Einrichtungen entwickelt, um eine möglichst homogene Verteilung einer flüssigen und einer gasförmigen Phase auf die Oberfläche eines Katalysatorfestbettes zu gewährleisten.

Die US 5,817,901 beschreibt ein Verfahren zur Selektivhydrierung von Kohlenwasserstoffschnitten mit 2 bis 20 Kohlenstoffatomen pro Molekül an einem Festbettkatalysator, wobei der Kohlenwasserstoffstrom und Wasserstoff auf das Festbett über einen statischen Mischer vom Typ SMV oder SMX der Firma Sulzer geleitet werden, vor den vorteilhaft ein nicht näher beschriebener Flüssigkeitsverteiler angeordnet ist. Beispielhaft werden Selektivhydrierungen von Butadien zu 1-Butenen aus einem C4-Schnitt für einen Reaktor mit einem Durchmesser von 10 cm beschrieben, wobei Selektivitäten für die obige Umsetzung von Butadien zu 1-Butenen von maximal etwa 58 % erreicht werden. Allein durch die Kombination eines statischen Mischers, beispielsweise vom SMV- oder SMX-Typ der Firma Sulzer und einem nicht näher spezifizierten Flüssigkeitsverteiler sind jedoch befriedigende Selektivitäten für die Selektivhydrierung von acetylenischen und dienischen Komponenten aus Kohlenwasserstoffschnitten in großtechnischen Reaktoren nicht erreichbar.

Die EP-A 1 147 809 beschreibt einen Verteiler für eine polyphasische Mischung, umfassend mindestens eine gasförmige und mindestens eine flüssige Phase, auf ein Festbett, gebildet aus einer Verteilerplatte, auf der Ablaufröhrchen angeordnet sind, und an deren unterem Ende ein weiteres Verteilerelement in Form eines Siebes vorgesehen ist.

Die WO-A 03/039733 beschreibt einen Verteiler für eine Mischung umfassend mindestens eine gasförmige und mindestens eine flüssige Phase, auf ein Festbett. Der Verteiler umfasst eine Verteilerplatte P, auf der mit einer Vielzahl von Misch- und Ablaufröhrchen durch die Platte, die eine obere Eintrittsöffnung für das Gas, seitliche Eintrittsöffnungen für die Flüssigkeit und gegebenenfalls einen kleinen Teil der gasförmigen Phase sowie eine untere Öffnung für die Mischung aus gasförmiger und flüssiger Phase aufweisen, wobei unterhalb der unteren Öffnung und oberhalb eines Katalysatorfestbettes ein zusätzliches Verteilerelement in Form eines Siebes mit kontrollierter Porosität und hochgebogenen Seitenwänden angeordnet ist. Durch die Anordnung des zusätzlichen Verteilerelementes wird eine gleichförmigere Beaufschlagung des Katalysatorfestbettes mit gasförmiger und flüssiger Phase im Vergleich zu einer Ausführungsform ohne dasselbe gewährleistet.

Die WO-A 95/35159 beschreibt einen weiteren Zweiphasenverteiler für eine gasförmige und eine flüssige Phase auf ein Festbett mit einer Verteilerplatte und Ablaufröhrchen, wobei zwei Gruppen von Ablaufröhrchen mit Öffnungen auf unterschiedlicher Höhe vorgesehen sind, dergestalt, dass eine gleichförmige Verteilung auch bei niedrigen Durchflussraten gewährleistet ist.

Die DE-A 10 2004 021 128 beschreibt einen weiteren Reaktor mit Zuleitung eines gasförmigen und eines flüssigen Eduktes im Gleichstrom zu einem Festbettkatalysator, wobei vor demselben ein Verteilerboden vorgesehen ist, der Öffnungen mit in den Öffnungen angeordneten Statikmischern aufweist. Durch die Kombination aus Verteilerboden und Statikmischern kann eine deutliche Verkleinerung des Schlankheitsgrades h/d, d. h. des Verhältnisses zwischen Höhe und Durchmesser des Reaktors, auf Werte von < 5, erreicht werden.

Mit den bekannten Verteilereinrichtungen waren jedoch Umsätze und Selektivitäten von Dreiphasenreaktionen, insbesondere von Selektivhydrierungen, bei großtechnischen Reaktoren, mit Durchmessern größer als 0,5 m, nicht befriedigend.

Es war daher Aufgabe der Erfindung, eine Misch- und Verteileinrichtung für einen Dreiphasenreaktor zur Verfügung zu stellen, die scale-up-fähig ist und die auch bei großtechnischen Reaktoren eine weitgehend gleichförmige Verteilung der gasförmigen und der flüssigen Phase und damit hohe Umsätze und Selektivitäten gewährleistet.

Die Aufgabe wird gelöst durch einen Reaktor gemäß Anspruch 1.

Erfindungsgemäß wird somit eine Misch- und Verteileinrichtung für eine flüssige und eine gasförmige Phase zur Verfügung gestellt, die zwei Hauptteile umfasst, einen Vorverteiler für die flüssige Phase, der analog bekannten Trogverteilern mit trogförmigen Kanälen ausgestattet ist, an die Ablaufröhrchen für die flüssige Phase angebracht sind, jedoch mit der Besonderheit, dass die Ablaufröhrchen soweit bis in die Nähe des darunter angeordneten Verteilerbodens, der als Hauptverteiler fungiert, reichen, dass die Ablaufröhrchen stets in die Flüssigkeit eintauchen, die auf dem Verteilerboden steht. Hierzu sind im Verteilerboden vertikale Tüllen eingebracht, mit einer oder mehreren Öffnungen für den Eintritt der gasförmigen Phase und einer oder mehreren unterhalb hiervon angeordneten Öffnungen für den Eintritt der flüssigen Phase, wobei die Anzahl und Größe der Öffnungen für den Eintritt der flüssigen Phase so ausgelegt sind, dass sich bei vorgegebenem Flüssigkeitszulauf der Flüssigkeitsspiegel auf dem Verteilerboden unterhalb der Öffnungen für den Eintritt der gasförmigen Phase und oberhalb der Öffnungen für den Eintritt der flüssigen Phase einstellt.

Indem somit die Ablaufröhrchen des Vorverteilers die Flüssigkeit stets bis unterhalb des Flüssigkeitsspiegels auf dem als Hauptverteiler fungierenden Verteilerboden reichen, wird die über die Ablaufröhrchen aus dem als Vorverteiler fungierenden Trogverteiler ablaufende Flüssigkeit gleichförmig in die auf dem Verteilerboden stehenden Flüssigkeit eingeleitet, ohne dass Flüssigkeit als Strahl austritt oder gar spritzt.

Bevorzugt wird die Anzahl und die Größe der Öffnungen für den Eintritt der flüssigen Phase in die Tüllen des Verteilerbodens dergestalt ausgelegt, dass sich der Flüssigkeitsspiegel auf dem Verteilerboden auch bei Abweichungen des Flüssigkeitszulaufs vom vorgegebenen Flüssigkeitszulauf um bis zu 20% nach oben und um bis zu 50% nach unten, unterhalb der Öffnungen für den Eintritt der gasförmigen Phase und oberhalb der Öffnungen für den Eintritt der flüssigen Phase einstellt.

Erfindungsgemäß wird eine gleichförmige Verteilung von flüssiger und gasförmiger Phase insbesondere auch für große Reaktoren mit einem Durchmesser im Bereich von 0,5 bis 5 m oder einem Bereich von 1 bis 4 m, oder auch in einem Bereich von 1,2 bis 3 m, gewährleistet.

Auf dem Verteilerboden, der als Hauptverteiler fungiert, sind 50 bis 200, oder bevorzugt 70 bis 150, oder auch 90 bis 130 Tüllen pro m² regelmäßig angeordnet.

Hierbei können die Tüllen in Dreiecksteilung oder in quadratischer Teilung auf dem Verteilerboden ausgerichtet sein.

Die Tüllen weisen einen freien Durchmesser von 5 bis 75 mm, eine Länge von 100 bis 600 mm oberhalb des Verteilerbodens und eine Länge von 20 bis 250 mm unterhalb des Verteilerbodens auf und haben Öffnungen für den Eintritt der flüssigen Phase mit einem Durchmesser von 2 bis 45 mm, die in einer Höhe von 10 bis 100 mm über dem Verteilerboden angeordnet sind, sowie Öffnungen für den Eintritt der gasförmigen Phase mit einem Durchmesser von 1 bis 30 mm.

Weiter bevorzugt weisen die Tüllen einen freien Durchmesser von 10 bis 60 mm, eine Länge von 200 bis 400 mm oberhalb der Verteilerbodens und eine Länge von 50 bis 200 mm unterhalb des Verteilerbodens auf, sowie Öffnungen für den Eintritt der flüssigen Phase mit einem Durchmesser von 3 bis 35 mm in einer Höhe von 22 bis 75 mm über dem Verteilerboden und Öffnungen für den Eintritt der gasförmigen Phase mit einem Durchmesser von 5 bis 20 mm.

Weiter bevorzugt haben die Tüllen einen freien Durchmesser von 35 bis 50 mm, eine Länge von 250 bis 350 mm oberhalb des Verteilerbodens und eine Länge von 100 bis 150 mm unterhalb des Verteilerbodens sowie Öffnungen für den Eintritt der flüssigen Phase mit einem Durchmesser von 10 bis 25 mm, die in einer Höhe von 40 bis 60 mm über dem Verteilerboden angeordnet sind und Öffnungen für den Eintritt der gasförmigen Phase mit einem Durchmesser von 5 bis 15 mm.

Vorteilhaft können die Öffnungen für den Eintritt der flüssigen Phase verstellbar in Höhe und Orientierung ausgebildet sein.

Bevorzugt wird der Druckverlust der gasförmigen Phase zwischen dem Eintritt in den Reaktor und dem Eintritt in die Tüllen über die Anzahl und Öffnungen für den Eintritt der gasförmigen Phase dergestalt gesteuert, dass bei der geringsten Gasbelastung im Reaktorbetrieb der Druckverlust der gasförmigen Phase zwischen dem Eintritt in den Reaktor und dem Eintritt in die Tüllen 50 bis 500 Pascal, bevorzugt 75 bis 300 Pascal, weiter bevorzugt 100 bis 200 Pascal, beträgt.

Der erfindungsgemäße Reaktor wird bevorzugt kontinuierlich betrieben.

Um bei notwendigen Betriebsunterbrechungen den Reaktor entleeren zu können, sind im Verteilerboden Leerlaufbohrungen vorgesehen, die gleichmäßig über den Verteilerboden verteilt sind und deren Anzahl und Größe dergestalt gewählt ist, dass im Normalbetrieb des Reaktors nur maximal 5% der gesamten, durch den Reaktor geleiteten flüssigen Phase durch die Leerlaufbohrungen durchfließt.

Bevorzugt haben die Leerlaufbohrungen einen Durchmesser zwischen 10 und 20 mm.

Der als Vorverteiler fungierende Trogverteiler, der trogförmige Kanäle mit Ablaufröhrchen in den trogförmigen Kanälen für die flüssige Phase aufweist, ist bevorzugt dergestalt ausgebildet, dass die trogförmigen Kanäle einen Hauptkanal und zwei oder mehrere Seitenkanäle umfassen, die so ausgebildet sind, dass die flüssige Phase ungehindert zwischen dem Hauptkanal und den Seitenkanälen strömen kann.

Insbesondere sind Hauptkanal und Seitenkanäle im Verhältnis zueinander dergestalt ausgebildet, dass die Summe der Grundfläche des Hauptkanals und der Seitenkanäle 30 bis 70%, bevorzugt 40 bis 60% der gesamten Querschnittsfläche des Reaktors beträgt.

Die trogförmigen Kanäle des Trogverteilers weisen bevorzugt eine Höhe von 200 bis 600 mm, besonders bevorzugt von 250 bis 500 mm, weiter bevorzugt von 300 bis 400 mm.

Am Boden der trogförmigen Kanäle sind bevorzugt 30 bis 120, weiter bevorzugt 40 bis 100, besonders bevorzugt 50 bis 80 Öffnungen pro m², mit einem Durchmesser von 5 bis 40, besonders bevorzugt von 10 bis 35, weiter bevorzugt von 15 bis 25 mm vorgesehen, wobei an jede Öffnung ein Ablaufröhrchen mit einem Durchmesser von 15 bis 75 mm, bevorzugt von 25 bis 60 mm, weiter bevorzugt von 35 bis 50 mm angebracht ist, und wobei die Länge der Ablaufröhrchen dergestalt eingestellt ist, dass das untere Ende derselben 20 bis 200 mm, bevorzugt 25 bis 150 mm oberhalb des Verteilerbodens liegt.

Eine weiter verbesserte Gleichverteilung wird durch eine bevorzugte Ausgestaltung des Reaktors erreicht, wonach die über ein Einlaufrohr in den Reaktor eingeleitete flüssige Phase am Ende des Einlaufrohres, an dem die flüssige Phase in den Reaktor eintritt, und vom Ende des Einlaufrohres um einige Zentimeter beabstandet, auf eine Prallplatte in Form einer Scheibe mit Öffnungen, auftrifft.

Gegenstand der Erfindung ist auch ein Verfahren gemäß Anspruch 17.

Gegenstand der Erfindung ist auch die Verwendung des vorstehend beschriebenen Reaktors oder des vorstehend beschriebenen Verfahrens zur Durchführung einer Selektivhydrierung von Kohlenwasserstoffschnitten. Bevorzugt ist eine Verwendung wonach die Kohlenwasserstoffschnitte C2-, C3- oder C4-Kohlenwasserstoffschnitte oder Pyrolysegase sind.

Besonders bevorzugt sind die Kohlenwasserstoffschnitte C4-Schnitte, wobei Butadien aus den C4-Schnitten selektiv zu n-Butenen hydriert wird. Durch den Einsatz des erfindungsgemäßen Reaktors mit spezieller Ausgestaltung des Vorverteilers und des Hauptverteilers ist es möglich, 1,3 Butadien weitgehend quantitativ zu n-Butenen zu hydrieren und dabei eine Überhydrierung zu Butan zu vermeiden.

Die Erfindung wird im Folgenden anhand einer Figur sowie von Ausführungsbeispielen näher erläutert.

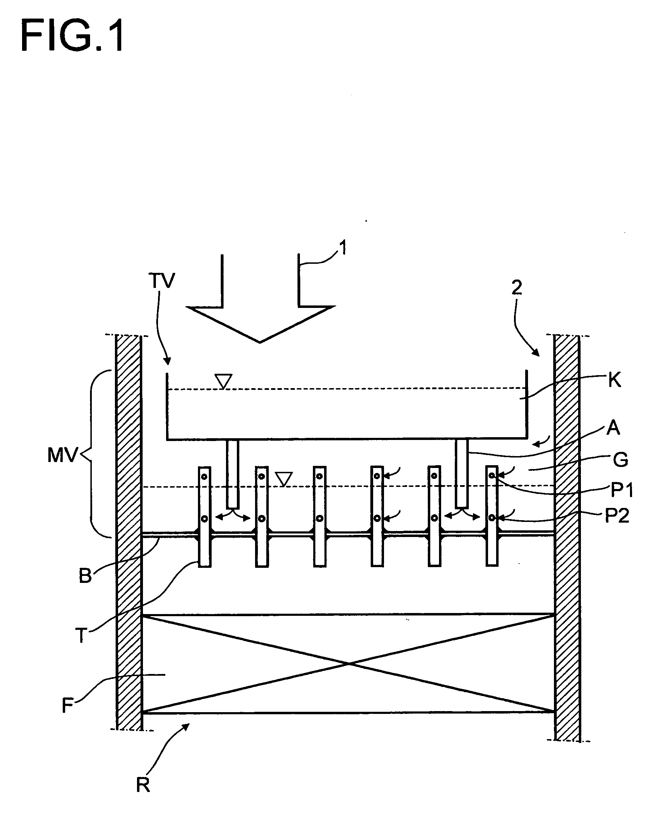

Die einzige Figur 1 zeigt die schematische Darstellung eines Längsschnitts durch einen erfindungsgemäßen Reaktor R mit Zuleitung der flüssigen Phase 1 und der gasförmigen Phase 2 jeweils von oben, über nicht dargestellte Stutzen.

Im Reaktor R ist eine Misch- und Verteileinrichtung MV oberhalb eines Katalysatorfestbettes F angeordnet, die einen Trogverteiler TV ausgebildeten Vorverteiler aufweist, mit am Boden desselben angeordneten Ablaufröhrchen A sowie einen Verteilerboden B, der stromabwärts des Trogverteilers TV angeordnet ist und als Hauptverteiler fungiert. Im Verteilerboden B sind Tüllen T eingebracht, die oberhalb und unterhalb des Verteilerbodens B in den Innenraum des Reaktors R ragen. Im Bereich der Tüllen T oberhalb des Verteilerbodens B sind Öffnungen P1 oberhalb des Flüssigkeitsspiegels auf dem Verteilerboden B, für den Eintritt der gasförmigen Phase 2, sowie Öffnungen P2 unterhalb des Flüssigkeitsspiegels auf dem Verteilerboden B, für den Eintritt der flüssigen Phase 1, vorgesehen.

Es wurde eine Misch- und Verteileinrichtung MV, wie sie schematisch in Figur 1 dargestellt ist, für einen Reaktor R mit einem Durchmesser von 1,7 m gebaut. Der Trogverteiler umfasste einen Hauptkanal und 6 Seitenkanäle mit einer Höhe von 400 mm, wobei die Summe der Grundflächen des Hauptkanals und der Seitenkanäle 60 % der gesamten Querschnittsfläche des Reaktors betrug. Am Boden der trogförmigen Kanäle waren 62 Öffnungen pro m², mit einem Durchmesser von 27 mm angeordnet, wobei an jede Öffnung ein Ablaufröhrchen mit einem Durchmesser von 48 mm angebracht war, und wobei die Länge der Ablaufröhrchen dergestalt gewählt war, dass das untere Ende derselben 150 mm oberhalb des Verteilerbodens lag.

Der Verteilerboden war mit 100 Tüllen pro m² in Dreiecksteilung bestückt, wobei die Tüllen einen freien Durchmesser von 41,9 mm, eine Länge von 320 mm oberhalb des Verteilerbodens und von 150 mm unterhalb des Verteilerbodens aufwiesen. In den Tüllen waren jeweils eine Öffnung, mit einem Durchmesser von 24 mm, in einer Höhe von 50 mm über dem Verteilerboden, für den Eintritt der flüssigen Phase sowie eine Öffnung mit einem Durchmesser von 15 mm in der Nähe des oberen Endes der Tüllen, für den Eintritt der gasförmigen Phase, vorgesehen.

Zur Beurteilung des Verteilverhaltens wurde die Misch- und Verteileinrichtung mit Wasser beaufschlagt. Die Wasserdurchflussmenge wurde zwischen 100 m³/h und 540 m³/h variiert.

Im gesamten obigen Lastbereich spritzte die Misch- und Verteileinrichtung nur wenig. Durch die Öffnungen für den Gaseintritt im oberen Bereich der Tüllen floss keine Flüssigkeit.

Ferner wurde die durch jede Tülle des Verteilerbodens fließende Wassermenge gemessen. Hierbei wurde festgestellt, dass die Durchflussmenge im Lastbereich von 300 bis 540 m³/h sehr wenig zwischen unterschiedlichen Tüllen variierte, mit etwas stärkerer Variation im Lastbereich unterhalb von 300 m³/h, wobei jedoch auch in diesem Lastbereich die Güte der Flüssigkeitsverteilung mit einer Standardabweichung kleiner 5% quantitativ hoch war.

Der oben beschriebene Reaktor mit Misch- und Verteileinrichtung wurde zur Selektivhydrierung von 1,3-Butadien aus einem C4-Schnitt zu n-Butenen eingesetzt.

Gegenüber der Durchführung des Verfahrens in einem Reaktor nach dem Stand der Technik stieg der Umsatz bezogen auf 1,3-Butadien um 1%, und die Selektivität für die Hydrierung des 1,3-Butadien zu n-Butenen um 3%.

## Patentansprüche

1. Reaktor (R) zur Durchführung einer Dreiphasenreaktion einer flüssigen Phase (1) und einer gasförmigen Phase (2) an einem Katalysatorfestbett (F), wobei das Katalysatorfestbett (F) horizontal im Reaktor (R) angeordnet ist und die flüssige Phase (1) und die gasförmige Phase (2) im Gleichstrom von oben nach unten über eine Misch- und Verteileinrichtung (MV) über das Katalysatorfestbett (F) durch den Reaktor (R) geleitet werden, wobei die Misch- und Verteileinrichtung (MV)
- einen Trogverteiler (TV) für die flüssige Phase (1) umfasst, mit trogförmigen Kanälen (K), und Ablaufröhrchen (A) in den trogförmigen Kanälen (K) für die flüssige Phase (1), sowie
- einen unterhalb des Trogverteilers (TV), von diesem beabstandet angeordneten Verteilerboden (B), in den vertikal Tüllen (T) eingebracht sind, **dadurch gekennzeichnet, dass** die Länge der Ablaufröhrchen (A) dergestalt eingestellt ist, dass das untere Ende derselben 20 bis 200 mm oberhalb des Verteilerbodens (B) liegt, und dass der Trogverteiler (TV) eine oder mehrere Öffnungen (P1) für den Eintritt der gasförmigen Phase (2) und eine oder mehrere Öffnungen (P2), die unterhalb der Öffnungen (P1) für den Eintritt der gasförmigen Phase angeordnet sind, für den Eintritt der flüssigen Phase (1) in die Tüllen (T) aufweist, und wobei die Anzahl und die Größe der Öffnungen (P2) für den Eintritt der flüssigen Phase (1) so ausgelegt sind, dass bei vorgegebenem Flüssigkeitszulauf der Flüssigkeitsspiegel auf dem Verteilerboden (B) unterhalb der Öffnungen (P1) für den Eintritt der gasförmigen Phase (2) und oberhalb der Öffnungen (P2) für den Eintritt der flüssigen Phase (1) einstellbar ist, und wobei stromaufwärts des Verteilerbodens (B) ein Stutzen angeordnet ist, über den die gasförmige Phase (2) in einen Gasraum (G) zwischen dem Druckverteiler (DV) und einem darunter beabstandet angeordneten Verteilerboden (B), in den vertikale Tüllen (T) eingebracht sind, eingeleitet wird, wobei auf dem Verteilerboden (B) 50 bis 200 Tüllen (T) pro m², regelmäßig angeordnet sind und wobei die Tüllen (T) einen freien Durchmesser von 5 bis 75 mm sowie eine Länge von 100 bis 600 mm oberhalb des Verteilerbodens (B) und eine Länge von 20 bis 250 mm unterhalb des Verteilerbodens (B) aufweisen, die Öffnungen (P2) für den Eintritt der flüssigen Phase (1) einen Durchmesser von 2 bis 45 mm aufweisen und in einer Höhe von 10 bis 100 mm über dem Verteilerboden (B) angeordnet sind und die Öffnungen (P1) für den Eintritt der gasförmigen Phase (2) einen Durchmesser von 1 bis 30 mm aufweisen.

2. Reaktor (R) nach Anspruch 1, **dadurch gekennzeichnet, dass** die Anzahl und die Größe der Öffnungen (P2) in den Tüllen (T) des Verteilerbodens (B) für den Eintritt der flüssigen Phase (1) dergestalt ausgelegt sind, dass sich der Flüssigkeitsspiegel auf dem Verteilerboden (B) auch bei Abweichungen des Flüssigkeitszulaufes vom vorgegebenen Flüssigkeitszulauf um bis zu 20% nach oben und um bis zu 50% nach unten, unterhalb der Öffnungen (P1) für den Eintritt der gasförmigen Phase (2) und oberhalb der Öffnungen (P2) für den Eintritt der flüssigen Phase (1) einstellt.

3. Reaktor (R) nach Anspruch 1 oder 2, **dadurch gekennzeichnet, dass** er einen Durchmesser im Bereich von 0,5 bis 5 m, bevorzugt in einem Bereich von 1 bis 4 m, weiter bevorzugt in einem Bereich von 1,2 bis 3 m, aufweist.

4. Reaktor (R) nach einem der Ansprüche 1 bis 3, **dadurch gekennzeichnet, dass** auf dem Verteilerboden (B) 70 bis 150, bevorzugt 90 bis 130 Tüllen (T) pro m², regelmäßig angeordnet sind.

5. Reaktor (R) nach Anspruch 4, **dadurch gekennzeichnet, dass** die Tüllen (T) in Dreiecksteilung oder in quadratischer Teilung auf dem Verteilerboden (B) angeordnet sind.

6. Reaktor (R) nach Anspruch 1, **dadurch gekennzeichnet, dass** die Tüllen (T) einen freien Durchmesser von 10 bis 60 mm sowie eine Länge von 200 bis 400 mm oberhalb des Verteilerbodens (B) und eine Länge von 50 bis 200 mm unterhalb des Verteilerbodens (B) aufweisen, und dass die Öffnungen (P2) für den Eintritt der flüssigen Phase (1) einen Durchmesser von 3 bis 35 mm aufweisen und in einer Höhe von 22 bis 75 mm über dem Verteilerboden (B) angeordnet sind und dass die Öffnungen (P1) für den Eintritt der gasförmigen Phase (2) einen Durchmesser von 5 bis 20 mm aufweisen.

7. Reaktor (R) nach Anspruch 6, **dadurch gekennzeichnet, dass** die Tüllen (T) einen freien Durchmesser von 35 bis 50 mm sowie eine Länge von 250 bis 350 mm oberhalb des Verteilerbodens (B) und eine Länge von 100 bis 150 mm unterhalb des Verteilerbodens (B) aufweisen, und dass die Öffnungen (P2) für den Eintritt der flüssigen Phase (1) einen Durchmesser von 10 bis 25 mm aufweisen und in einer Höhe von 40 bis 60 mm über dem Verteilerboden (B) angeordnet sind und dass die Öffnungen (P1) für den Eintritt der gasförmigen Phase (2) einen Durchmesser von 7 bis 15 mm aufweisen.

8. Reaktor (R) nach einem der Ansprüche 1 bis 7, **dadurch gekennzeichnet, dass** die Öffnungen (P2) für den Eintritt der flüssigen Phase (1) verstellbar in Höhe und Orientierung ausgebildet sind.

9. Reaktor (R) nach einem der Ansprüche 1 bis 8, **dadurch gekennzeichnet, dass** im Verteilerboden (B) Leerlaufbohrungen vorgesehen sind, die gleichmäßig über den Verteilerboden (B) verteilt sind und deren Anzahl und Größe dergestalt gewählt ist, dass im Normalbetrieb nur maximal 5 % der gesamten, durch den Reaktor (R) geleiteten flüssigen Phase (1) durch die Leerlaufbohrungen durchfließt.

10. Reaktor (R) nach Anspruch 9, **dadurch gekennzeichnet, dass** die Leerlaufbohrungen einen Durchmesser zwischen 10 und 20 mm aufweisen.

11. Reaktor (R) nach einem der Ansprüche 1 bis 10, **dadurch gekennzeichnet, dass** der Trogverteiler (TV) 10 bis 300 mm, bevorzugt 20 bis 200 mm oberhalb des oberen Endes der Tüllen (T) angeordnet ist.

12. Reaktor (R) nach einem der Ansprüche 1 bis 11, **dadurch gekennzeichnet, dass** die trogförmigen Kanäle (K) des Trogverteilers (TV) einen Hauptkanal und zwei oder mehrere Seitenkanäle umfassen, die dergestalt ausgebildet sind, dass die flüssige Phase (1) ungehindert zwischen dem Hauptkanal und den Seitenkanälen strömen kann.

13. Reaktor (R) nach einem der Ansprüche 1 bis 12, **dadurch gekennzeichnet, dass** die Summe der Grundflächen des Hauptkanals und der Seitenkanäle 30 bis 70 %, bevorzugt 40 bis 60% der gesamten Querschnittsfläche des Reaktors (R) beträgt.

14. Reaktor (R) nach einem der Ansprüche 1 bis 13, **gekennzeichnet durch** eine Höhe der trogförmigen Kanäle (K) von 200 bis 600 mm, bevorzugt von 250 bis 500 mm, weiter bevorzugt von 300 bis 400 mm.

15. Reaktor (R) nach einem der Ansprüche 1 bis 14, **dadurch gekennzeichnet, dass** am Boden der trogförmigen Kanäle (K) 30 bis 120, bevorzugt 40 bis 100, weiter bevorzugt 50 bis 80 Öffnungen pro m², mit einem Durchmesser von 5 bis 40, bevorzug von 10 bis 35, weiter bevorzugt von 15 bis 25 mm vorgesehen sind und dass an jeder Öffnung ein Ablaufröhrchen (A) mit einem Durchmesser von 15 bis 75 mm, bevorzugt von 25 bis 60 mm, weiter bevorzugt von 35 bis 50, mm angebracht ist, wobei die Länge der Ablaufröhrchen (A) dergestalt eingestellt ist, dass das untere Ende derselben 25 bis 150 mm oberhalb des Verteilerbodens (B) liegt.

16. Reaktor (R) nach einem der Ansprüche 1 bis 15, **dadurch gekennzeichnet, dass** die flüssige Phase (1) über ein Einlaufrohr in den Reaktor (R) eingeleitet wird und dass am Ende des Einlaufrohres, an dem die flüssige Phase (1) in den Reaktor (R) eintritt, vom Ende des Einlaufrohres um einige cm beabstandet, eine Prallplatte in Form einer Scheibe mit Öffnungen angeordnet ist.

17. Verfahren zur Durchführung einer Dreiphasenreaktion in einem Reaktor (R) zwischen einer flüssigen Phase (1) und einer gasförmigen Phase (2) an einem Katalysatorfestbett (F), wobei das Katalysatorfestbett (F) horizontal im Reaktor (R) angeordnet ist und die flüssige Phase (1) und die gasförmige Phase (2) im Gleichstrom von oben nach unten über eine Misch- und Verteileinrichtung (MV) über das Festbett (F) durch den Reaktor (R) geleitet werden, **dadurch gekennzeichnet, dass**
- die flüssige Phase (1) von außerhalb des Reaktors (R) über ein Einlaufrohr in einen Trogverteiler (TV), mit trogförmigen Kanälen (K) und Ablaufröhrchen (A) in den trogförmigen Kanälen (K) und
- die gasförmige Phase (2) getrennt und/oder gemeinsam mit der flüssigen Phase (1) über einen Stutzen, der stromaufwärts des Verteilerbodens (B) angeordnet ist, in einen Gasraum (G) zwischen dem Trogverteiler (TV) und einem darunter beabstandet angeordneten Verteilerboden (B), in den vertikale Tüllen (T) eingebracht sind, eingeleitet wird, wobei
- die flüssige Phase (1) über die Ablaufröhrchen (A) des Trogverteilers (TV) unterhalb des Flüssigkeitsspiegels in die auf dem Verteilerboden (B) stehende Flüssigkeit und
- die gasförmige Phase (2) über eine oder mehrere Öffnungen (P1) aus dem Gasraum (G) in die Tüllen (T) des Verteilerbodens (B) eingeleitet wird und wobei
- die flüssige Phase (1) über eine oder mehrere Öffnungen (P2) unterhalb des Flüssigkeitsspiegels der Flüssigkeit in die Tüllen (T) auf dem Verteilerboden (B) und die Mischung aus flüssiger Phase (1) und gasförmiger Phase (2) über die Tüllen (T) auf das Katalysatorfestbett (F) geleitet wird, wobei die Länge der Ablaufröhrchen (A) dergestalt eingestellt ist, dass das untere Ende derselben 20 bis 200 mm oberhalb des Verteilerbodens (B) liegt und wobei auf dem Verteilerboden (B) 50 bis 200 Tüllen (T) pro m², regelmäßig angeordnet sind und wobei die Tüllen (T) einen freien Durchmesser von 5 bis 75 mm sowie eine Länge von 100 bis 600 mm oberhalb des Verteilerbodens (B) und eine Länge von 20 bis 250 mm unterhalb des Verteilerbodens (B) aufweisen, die Öffnungen (P2) für den Eintritt der flüssigen Phase (1) einen Durchmesser von 2 bis 45 mm aufweisen und in einer Höhe von 10 bis 100 mm über dem Verteilerboden (B) angeordnet sind und die Öffnungen (P1) für den Eintritt der gasförmigen Phase (2) einen Durchmesser von 1 bis 30 mm aufweisen.

18. Verwendung des Reaktors nach einem der Ansprüche 1 bis 16 oder des Verfahrens nach Anspruch 18 zur Durchführung einer Selektivhydrierung von Kohlenwasserstoffschnitten.

19. Verwendung nach Anspruch 18, **dadurch gekennzeichnet, dass** die Kohlenwasserstoffschnitte C2-, C3- oder C4-Kohlenwasserstoffschnitte oder Pyrolysegase sind.

20. Verwendung nach Anspruch 19 **dadurch gekennzeichnet, dass** die Kohlenwasserstoffschnitte C4-Schnitte sind und dass Butadien aus den C4-Schnitten selektiv zu n-Butenen hydriert wird.

## Claims

1. A reactor (R) for carrying out a three-phase reaction of a liquid phase (1) and a gaseous phase (2) over a fixed catalyst bed (F), where the fixed catalyst bed (F) is arranged horizontally in the reactor (R) and the liquid phase (1) and the gaseous phase (2) are passed through the reactor (R) in cocurrent from the top downward via a mixing and distribution device (MD) over the fixed catalyst bed (F), where the mixing and distribution device (MD) comprises
- a trough distributor (TD) for the liquid phase (1) having trough-shaped channels (C) and outlet tubes (O) in the trough-shaped channels (C) for the liquid phase (1) and
- a distributor plate (P) which is arranged with a spacing below the trough distributor (TD) and in which vertical nozzles (N) are arranged, wherein the length of the outlet tubes (O) is set so that the lower end of these is from 20 to 200 mm above the distributor plate (P) and the trough distributor (TD) has one or more openings (P1) for entry of the gaseous phase (2) and one or more openings (P2), which are arranged below the openings (P1) for entry of the gaseous phase, for entry of the liquid phase (1) into the nozzles (N) and the number and size of the openings (P2) for entry of the liquid phase (1) is designed so that, at a predetermined liquid feed rate, the surface of the liquid on the distributor plate (P) can be set below the openings (P1) for entry of the gaseous phase (2) and above the openings (P2) for entry of the liquid phase (1) and a port via which the gaseous phase (2) is introduced into a gas space (G) between the pressure distributor (PD) and a distributor plate (P) which is arranged with a spacing underneath and in which vertical nozzles (N) are installed is arranged upstream of the distributor plate (P), where from 50 to 200 nozzles (N) per m² are arranged regularly on the distributor plate (P) and the nozzles (N) have a free diameter of from 5 to 75 mm and a length of from 100 to 600 mm above the distributor plate (P) and a length of from 20 to 250 mm below the distributor plate (P), the openings (P2) for entry of the liquid phase (1) have a diameter of from 2 to 45 mm and are arranged at a height of from 10 to 100 mm above the distributor plate (P) and the openings (P1) for entry of the gaseous phase (2) have a diameter of from 1 to 30 mm.

2. The reactor (R) according to claim 1, wherein the number and size of the openings (P2) in the nozzles (N) of the distributor plate (P) for entry of the liquid phase (1) are designed so that the surface of the liquid on the distributor plate (P) is established below the openings (P1) for entry of the gaseous phase (2) and above the openings (P2) for entry of the liquid phase (1) even in the case of deviations of the liquid feed rate from the predetermined liquid feed rate by up to 20% in an upward direction and by up to 50% in a downward direction.

3. The reactor (R) according to claim 1 or 2 which has a diameter in the range from 0.5 to 5 m, preferably in the range from 1 to 4 m, more preferably in the range from 1.2 to 3 m.

4. The reactor (R) according to any of claims 1 to 3, wherein from 70 to 150, preferably from 90 to 130, nozzles (N) per m² are arranged regularly on the distributor plate (P).

5. The reactor (R) according to claim 4, wherein the nozzles (N) are arranged in triangular positions or in a square arrangement on the distributor plate (P).

6. The reactor (R) according to claim 1, wherein the nozzles (N) have a free diameter of from 10 to 60 mm and a length of from 200 to 400 mm above the distributor plate (P) and a length of from 50 to 200 mm below the distributor plate (P) and the openings (P2) for entry of the liquid phase (1) have a diameter of from 3 to 35 mm and are arranged at a height of from 22 to 75 mm above the distributor plate (P) and the openings (P1) for entry of the gaseous phase (2) have a diameter of from 5 to 20 mm.

7. The reactor (R) according to claim 6, wherein the nozzles (N) have a free diameter of from 35 to 50 mm and a length of from 250 to 350 mm above the distributor plate (P) and a length of from 100 to 150 mm below the distributor plate (P) and the openings (P2) for entry of the liquid phase (1) have a diameter of from 10 to 25 mm and are arranged at a height of from 40 to 60 mm above the distributor plate (P) and the openings (P1) for entry of the gaseous phase (2) have a diameter of from 7 to 15 mm.

8. The reactor (R) according to any of claims 1 to 7, wherein the openings (P2) for entry of the liquid phase (1) can be adjusted in respect of their height and orientation.

9. The reactor (R) according to any of claims 1 to 8, wherein the distributor plate (P) is provided with no-load holes which are distributed uniformly over the distributor plate (P) and whose number and size is selected so that in normal operation only a maximum of 5% of the total liquid phase (1) passed through the reactor (R) flows through the no-load holes.

10. The reactor (R) according to claim 9, wherein the no-load holes have a diameter of from 10 to 20 mm.

11. The reactor (R) according to any of claims 1 to 10, wherein the trough distributor (TD) is arranged from 10 to 300 mm, preferably from 20 to 200 mm, above the upper end of the nozzles (N).

12. The reactor (R) according to any of claims 1 to 11, wherein the trough-shaped channels (C) of the trough distributor (TD) comprise a main channel and two or more side channels which are configured so that the liquid phase (1) can flow unhindered between the main channel and the side channels.

13. The reactor (R) according to any of claims 1 to 12, wherein the sum of the base areas of the main channel and the side channels is from 30 to 70%, preferably from 40 to 60%, of the total cross-sectional area of the reactor (R).

14. The reactor (R) according to any of claims 1 to 13 in which the height of the trough-shaped channels (C) is from 200 to 600 mm, preferably from 250 to 500 mm, more preferably from 300 to 400 mm.

15. The reactor (R) according to any of claims 1 to 14, wherein from 30 to 120, preferably from 40 to 100, more preferably from 50 to 80, openings per m² having a diameter of from 5 to 40 mm, preferably from 10 to 35 mm, more preferably from 15 to 25 mm, are provided at the bottom of the trough-shaped channels (C) and an outlet tube (O) having a diameter of from 15 to 75 mm, preferably from 25 to 60 mm, more preferably from 35 to 50 mm, is installed at each opening, with the length of the outlet tubes (O) being set so that the lower end of these is from 25 to 150 mm above the distributor plate (P).

16. The reactor (R) according to any of claims 1 to 15, wherein the liquid phase (1) is introduced into the reactor (R) via an inlet tube and an impingement plate in the form of a disk with openings is arranged at the end of the inlet tube at which the liquid phase (1) enters the reactor (R) at a distance of a few cm from the end of the inlet tube.

17. A process for carrying out a three-phase reaction between a liquid phase (1) and a gaseous phase (2) over a fixed catalyst bed (F) in a reactor (R), with the fixed catalyst bed (F) being arranged horizontally in the reactor (R) and the liquid phase (1) and the gaseous phase (2) being passed through the reactor (R) in cocurrent from the top downward via a mixing and distribution device (MD) over the fixed catalyst bed (F), wherein
- the liquid phase (1) is introduced from outside the reactor (R) via an inlet tube into a trough distributor (TD) having trough-shaped channels (C) and outlet tubes (O) in the trough-shaped channels (C) and
- the gaseous phase (2) is introduced separately and/or together with the liquid phase (1) via a port which is arranged upstream of the distributor plate (P) into a gas space (G) between the trough distributor (TD) and a distributor plate (P) which is arranged with a spacing below the trough distributor (TD) and in which vertical nozzles (N) are installed, where
- the liquid phase (1) is introduced via the outlet tubes (O) of the trough distributor (TD) below the surface of the liquid into the liquid standing on the distributor plate (P) and
- the gaseous phase (2) is introduced via one or more openings (P1) from the gas space (G) into the nozzles (N) of the distributor plate (P) and
- the liquid phase (1) is fed via one or more openings (P2) below the surface of the liquid into the nozzles (N) on the distributor plate (P) and the mixture of liquid phase (1) and gaseous phase (2) being fed via the nozzles (N) to the fixed catalyst bed (F), where the length of the output tubes (O) is set so that the lower end of these is from 20 to 200 mm above the distributor plate (P) and from 50 to 200 nozzles (N) per m² are arranged regularly on the distributor plate (P) and the nozzles (N) have a free diameter of from 5 to 75 mm and a length of from 100 to 600 mm above the distributor plate (P) and a length of from 20 to 250 mm below the distributor plate (P), the openings (P2) for entry of the liquid phase (1) have a diameter of from 2 to 45 mm and are arranged at a height of from 10 to 100 mm above the distributor plate (P) and the openings (P1) for entry of the gaseous phase (2) have a diameter of from 1 to 30 mm.

18. The use of the reactor according to any of claims 1 to 16 or the process according to claim 18 for carrying out a selective hydrogenation of hydrocarbon fractions.

19. The use according to claim 18, wherein the hydrocarbon fractions are C2, C3 or C4 hydrocarbon fractions or pyrolysis gases.

20. The use according to claim 19, wherein the hydrocarbon fractions are C4 fractions and butadiene in the C4 fractions is selectively hydrogenated to n-butenes.

## Revendications

1. Réacteur (R) pour la réalisation d'une réaction triphasée d'une phase liquide (1) et d'une phase gazeuse (2) sur un lit fixe catalytique (F), le lit fixe catalytique (F) étant agencé horizontalement dans le réacteur (R), et la phase liquide (1) et la phase gazeuse (2) étant conduites dans le réacteur (R) à co-courant du haut vers le bas par le biais d'un dispositif de mélange et de distribution (MV) en passant sur le lit fixe catalytique (F), le dispositif de mélange et de distribution (MV)
- comprenant un distributeur à auge (TV) pour la phase liquide (1), muni de canaux en forme d'auge (K) et de tubes d'écoulement (A) dans les canaux en forme d'auge (K) pour la phase liquide (1), et
- un fond de distributeur (B) placé sous le distributeur à auge (TV), espacé de celui-ci, dans lequel des becs verticaux (T) sont disposés, **caractérisé en ce que** la longueur des tubes d'écoulement (A) est ajustée de manière à ce que leur extrémité inférieure se situe 20 à 200 mm au-dessus du fond de distributeur (B), et **en ce que** le distributeur à auge (TV) comprend une ou plusieurs ouvertures (P1) pour l'entrée de la phase gazeuse (2) et une ou plusieurs ouvertures (P2), qui sont placées sous les ouvertures (P1) pour l'entrée de la phase gazeuse, pour l'entrée de la phase liquide (1) dans les becs (T), le nombre et la taille des ouvertures (P2) pour l'entrée de la phase liquide (1) étant configurés de manière à ce que, pour une alimentation de liquide prédéterminée, le niveau de liquide sur le fond de distributeur (B) puisse être ajusté en dessous des ouvertures (P1) pour l'entrée de la phase gazeuse (2) et au-dessus des ouvertures (P2) pour l'entrée de la phase liquide (1), et une tubulure étant placée en amont du fond de distributeur (B), par laquelle la phase gazeuse (2) est introduite dans un espace de tête (G) entre le distributeur sous pression (DV) et un fond de distributeur (B) placé en dessous, espacé de celui-ci, dans lequel des becs verticaux (T) sont disposés, 50 à 200 becs (T) par m² étant agencés régulièrement sur le fond de distributeur (B) et les becs (T) présentant un diamètre libre de 5 à 75 mm, ainsi qu'une longueur de 100 à 600 mm au-dessus du fond de distributeur (B) et une longueur de 20 à 250 mm en dessous du fond de distributeur (B), les ouvertures (P2) pour l'entrée de la phase liquide (1) présentant un diamètre de 2 à 45 mm et étant placées à une hauteur de 10 à 100 mm au-dessus du fond de distributeur (B), et les ouvertures (P1) pour l'entrée de la phase gazeuse (2) présentant un diamètre de 1 à 30 mm.

2. Réacteur (R) selon la revendication 1, **caractérisé en ce que** le nombre et la taille des ouvertures (P2) dans les becs (T) du fond de distributeur (B) pour l'entrée de la phase liquide (1) sont configurés de manière à ce que le niveau de liquide sur le fond de distributeur (B) soit ajusté en dessous des ouvertures (P1) pour l'entrée de la phase gazeuse (2) et au-dessus des ouvertures (P2) pour l'entrée de la phase liquide (1), même en cas de fluctuations de l'alimentation de liquide de jusqu'à 20 % au-dessus et de jusqu'à 50 % en dessous de l'alimentation de liquide prédéterminée.

3. Réacteur (R) selon la revendication 1 ou 2, **caractérisé en ce qu'**il présente un diamètre dans la plage allant de 0,5 à 5 m, de préférence dans une plage allant de 1 à 4 m, de manière davantage préférée dans une plage allant de 1,2 à 3 m.

4. Réacteur (R) selon l'une quelconque des revendications 1 à 3, **caractérisé en ce que** 70 à 150, de préférence 90 à 130 becs (T) par m² sont agencés régulièrement sur le fond de distributeur (B).

5. Réacteur (R) selon la revendication 4, **caractérisé en ce que** les becs (T) sont agencés selon une distribution triangulaire ou quadratique sur le fond de distributeur (B).

6. Réacteur (R) selon la revendication 1, **caractérisé en ce que** les becs (T) présentent un diamètre libre de 10 à 60 mm, ainsi qu'une longueur de 200 à 400 mm au-dessus du fond de distributeur (B) et une longueur de 50 à 200 mm en dessous du fond de distributeur (B), et **en ce que** les ouvertures (P2) pour l'entrée de la phase liquide (1) présentent un diamètre de 3 à 35 mm et sont placées à une hauteur de 22 à 75 mm au-dessus du fond de distributeur (B), et **en ce que** les ouvertures (P1) pour l'entrée de la phase gazeuse (2) présentent un diamètre de 5 à 20 mm.

7. Réacteur (R) selon la revendication 6, **caractérisé en ce que** les becs (T) présentent un diamètre libre de 35 à 50 mm, ainsi qu'une longueur de 250 à 350 mm au-dessus du fond de distributeur (B) et une longueur de 100 à 150 mm en dessous du fond de distributeur (B), et **en ce que** les ouvertures (P2) pour l'entrée de la phase liquide (1) présentent un diamètre de 10 à 25 mm et sont placées à une hauteur de 40 à 60 mm au-dessus du fond de distributeur (B), et **en ce que** les ouvertures (P1) pour l'entrée de la phase gazeuse (2) présentent un diamètre de 7 à 15 mm.

8. Réacteur (R) selon l'une quelconque des revendications 1 à 7, **caractérisé en ce que** les ouvertures (P2) pour l'entrée de la phase liquide (1) sont conçues sous une forme ajustable en hauteur et en orientation.

9. Réacteur (R) selon l'une quelconque des revendications 1 à 8, **caractérisé en ce que** des orifices de déversement libres sont prévus dans le fond de distributeur (B), qui sont répartis régulièrement sur le fond de distributeur (B) et dont le nombre et la taille sont choisis de manière à ce qu'en fonctionnement normal, seulement au plus 5 % de la totalité de la phase liquide (1) introduite dans le réacteur (R) s'écoule dans les orifices de déversement libres.

10. Réacteur (R) selon la revendication 9, **caractérisé en ce que** les orifices de déversement libres présentent un diamètre compris entre 10 et 20 mm.

11. Réacteur (R) selon l'une quelconque des revendications 1 à 10, **caractérisé en ce que** le distributeur à auge (TV) est placé 10 à 300 mm, de préférence 20 à 200 mm, au-dessus de l'extrémité supérieure des becs (T).

12. Réacteur (R) selon l'une quelconque des revendications 1 à 11, **caractérisé en ce que** les canaux en forme d'auge (K) du distributeur à auge (TV) comprennent un canal principal et deux canaux latéraux ou plus, qui sont conçus de manière à ce que la phase liquide (1) puisse s'écouler sans encombre entre le canal principal et les canaux latéraux.

13. Réacteur (R) selon l'une quelconque des revendications 1 à 12, **caractérisé en ce que** la somme des surfaces de base du canal principal et des canaux latéraux est de 30 à 70 %, de préférence 40 à 60 %, de la totalité de la surface de section transversale du réacteur (R).

14. Réacteur (R) selon l'une quelconque des revendications 1 à 13, **caractérisé par** une hauteur des canaux en forme d'auge (K) de 200 à 600 mm, de préférence de 250 à 500 mm, de manière davantage préférée de 300 à 400 mm.

15. Réacteur (R) selon l'une quelconque des revendications 1 à 14, **caractérisé en ce que** 30 à 120, de préférence 40 à 100, de manière davantage préférée 50 à 80 ouvertures par m², d'un diamètre de 5 à 40, de préférence de 10 à 35, de manière davantage préférée de 15 à 25 mm, sont prévues au fond des canaux en forme d'auge (K) , et **en ce qu'**un tube d'écoulement (A) d'un diamètre de 15 à 75 mm, de préférence de 25 à 60 mm, de manière davantage préférée de 35 à 50 mm, est fixé sur chaque ouverture, la longueur des tubes d'écoulement (A) étant ajustée de manière à ce que leur extrémité inférieure se situe 25 à 150 mm au-dessus du fond de distributeur (B).

16. Réacteur (R) selon l'une quelconque des revendications 1 à 15, **caractérisé en ce que** la phase liquide (1) est introduite dans le réacteur (R) par le biais d'un tube d'entrée et **en ce qu'**un déflecteur sous la forme d'un disque muni d'ouvertures est placé à l'extrémité du tube d'entrée, au niveau de laquelle la phase liquide (1) entre dans le réacteur (R), espacé de quelques cm de l'extrémité du tube d'entrée.

17. Procédé de réalisation d'une réaction triphasée dans un réacteur (R) entre une phase liquide (1) et une phase gazeuse (2) sur un lit fixe catalytique (F), le lit fixe catalytique (F) étant agencé horizontalement dans le réacteur (R), et la phase liquide (1) et la phase gazeuse (2) étant conduites dans le réacteur (R) à co-courant du haut vers le bas par le biais d'un dispositif de mélange et de distribution (MV) en passant sur le lit fixe (F), **caractérisé en ce que**
- la phase liquide (1) est introduite depuis l'extérieur du réacteur (R) par le biais d'un tube d'entrée dans un distributeur à auge (TV), muni de canaux en forme d'auge (K) et de tubes d'écoulement (A) dans les canaux en forme d'auge (K), et
- la phase gazeuse (2) est introduite séparément et/ou avec la phase liquide (1) par le biais d'une tubulure, qui est placée en amont du fond de distributeur (B), dans un espace de tête (G) entre le distributeur à auge (TV) et un fond de distributeur (B) placé en dessous, espacé de celui-ci, dans lequel des becs verticaux (T) sont disposés,
- la phase liquide (1) étant introduite par le biais des tubes d'écoulement (A) du distributeur à auge (TV) sous le niveau de liquide dans le liquide se trouvant sur le fond de distributeur (B) et
- la phase gazeuse (2) étant introduite par le biais d'une ou de plusieurs ouvertures (P1) depuis l'espace de tête (G) dans les becs (T) du fond de distributeur (B), et
- la phase liquide (1) étant introduite par une ou plusieurs ouvertures (P2) en dessous du niveau de liquide du liquide dans les becs (T) sur le fond de distributeur (B) et le mélange de la phase liquide (1) et de la phase gazeuse (2) étant conduit par les becs (T) sur le lit fixe catalytique (F), la longueur des tubes d'écoulement (A) étant ajustée de manière à ce que leur extrémité inférieure se situe 20 à 200 mm au-dessus du fond de distributeur (B), et 50 à 200 becs (T) par m² étant agencés régulièrement sur le fond de distributeur (B), et les becs (T) présentant un diamètre libre de 5 à 75 mm, ainsi qu'une longueur de 100 à 600 mm au-dessus du fond de distributeur (B) et une longueur de 20 à 250 mm en dessous du fond de distributeur (B), les ouvertures (P2) pour l'entrée de la phase liquide (1) présentant un diamètre de 2 à 45 mm et étant placées à une hauteur de 10 à 100 mm au-dessus du fond de distributeur (B), et les ouvertures (P1) pour l'entrée de la phase gazeuse (2) présentant un diamètre de 1 à 30 mm.

18. Utilisation du réacteur selon l'une quelconque des revendications 1 à 16 ou du procédé selon la revendication 18 pour la réalisation d'une hydrogénation sélective de coupes d'hydrocarbures.

19. Utilisation selon la revendication 18, **caractérisée en ce que** les coupes d'hydrocarbures sont des coupes d'hydrocarbures en C2, C3 ou C4 ou des gaz de pyrolyse.

20. Utilisation selon la revendication 19, **caractérisée en ce que** les coupes d'hydrocarbures sont des coupes en C4 et **en ce que** le butadiène des coupes en C4 est hydrogéné sélectivement en n-butènes.
